# FASCICULE DE BREVET EUROPEEN

(11) **EP 0 780 120 B1**
(45) Date de publication et mention de la délivrance du brevet: **08.07.1998**
(21) Numéro de dépôt: 96402451.7
(22) Date de dépôt: 15.11.1996
(51) Int. Cl.: A61K 7/48

(54) **Utilisation de la procystéine comme agent dépigmentant**
Verwendung von Procystein als depigmentierendes Mittel
Use of procysteine as skin whitening agent

(30) Priorité: 22.12.1995 FR 9515335
(43) Date de publication de la demande: 25.06.1997
(73) Titulaire: L'OREAL, 75008 Paris (FR)
(72) Inventeur: Galey, Jean-Baptiste, 93600 Aulnay-sous-Bois (FR); Marrot, Laurent, 93190 Livry Gargan (FR); Causse, Catherine, 92340 Bourg La Reine (FR); Regnier, Marcelle, 75018 Paris (FR); Schmidt, Rainer, 75018 Paris (FR)
(74) Mandataire: Lhoste, Catherine

(56) Documents cités:
- EP-A- 0 117 176
- EP-A- 0 650 725
- US-A- 5 208 249

## Description

La présente invention se rapporte à une utilisation de l'acide L-2-oxothiazolidine 4-carboxylique comme agent dépigmentant ou blanchissant dans une composition cosmétique et/ou dermatologique.

La couleur de la peau est fonction de différents facteurs et notamment des saisons de l'année, de la race et du sexe, et elle est principalement déterminée par la concentration de mélanine produite par les mélanocytes.

Par contre depuis plusieurs années, on cherche à diminuer et/ou ralentir cette production de mélanine en vue de dépigmenter ou blanchir la peau en agissant sur un ou plusieurs des points de la synthèse biochimique intracellulaire de la mélanine.

Aussi depuis un bon nombre d'années, différentes molécules ont déjà été utilisées et testées comme agent dépigmentant ou blanchissant.

Notamment, on a déjà incorporé, dans des compositions, des composés tels que la vitamine C, les dérivés de vitamine C ou de vitamine E, l'arbutine, l'hydroquinone, l'acide kojique, les dérivés placentaires, le gluthation et ses dérivés.

Ces différents composés sont connus pour agir sur la synthèse et/ou l'activité de la tyrosinase, enzyme qui entre en jeu dans la synthèse de la mélanine, ou pour réduire la quantité de mélanine formée ou encore pour stimuler l'élimination de la mélanine au travers des kératinocytes. Malheureusement, ils sont soit toxiques, cas de l'hydroquinone, pour être appliqués sur la peau, soit instables en solution, cas de la vitamine C et de l'acide kojique ce qui complique quelque peu la fabrication de la composition, soit encore peuvent présenter des odeurs désagréables et notamment des odeurs soufrées en ce qui concerne le gluthation, ce qui en limite par conséquent l'utilisation. De plus le nombre de ces composés est très limité.

Aussi, il subsiste le besoin d'un nouvel agent blanchissant de la peau à action aussi efficace que ceux connus mais n'ayant pas leurs inconvénients, c'est-à-dire qui soit stable dans une composition, non toxique pour la peau et qui ne présente pas d'odeurs désagréable et ce surtout dans la mesure où cet agent est appliqué sur la peau.

Certes il est connu d'utiliser l'acide L-2-oxothiazolidine 4-carboxylique encore appelé "procystéine" comme agent antichute (document EP 656 201). Toutefois personne jusqu'à ce jour n'avait envisagé de l'utiliser comme agent dépigmentant.

L'invention a donc pour objet une utilisation de l'acide L-2-oxothiazolidine 4-carboxylique dans une compositon cosmétique comme agent dépigmentant et/ou blanchissant.

L'invention a aussi pour objet une utilisation de l'acide L-2-oxothiazolidine 4-carboxylique pour la fabrication d'une composition dermatologique destinée à la dépigmentation et/ou au blanchiment de la peau.

Au contact de la peau, cet acide procure l'avantage de ne pas générer un dégagement d'odeur soufrée désagréable.

Cet acide peut être présent en une quantité allant de 0,1 à 10 % en poids et de préférence de 2 à 5 % en poids par rapport au poids total de la composition.

La composition selon l'invention peut de plus comprendre tous les ingrédients classiquement utilisés dans le domaine cosmétique ou dermatologique, dans des concentrations habituelles. Ces ingrédients sont en particuliers choisi parmi les corps gras, les conservateurs, les vitamines, les gélifiants, les parfums, les tensioactifs, l'eau, les antioxydants, les charges, les hydratants, les filtres et leurs mélanges.

Parmi les corps gras, on peut choisir une huile minérale ou synthétique, une cire, une silicone, un alcool gras ou un acide gras. L'huile peut être choisie parmi l'huile de vaseline, l'huile de jojoba, et la cire parmi la cire de sipol. Ces huiles et ces cires peuvent être d'origine naturelle ou syntéthique.

Les tensioactifs peuvent être choisis parmi le mono diglycéryl stéarate de sodium, le stéarate de sodium, et les gélifiants parmi les polyéthylènes glycol éventuellement oxyéthylénés.

La composition de l'invention peut se présenter sous forme de solution aqueuse, hydroalcoolique ou huileuse, d'émulsion huile-dans-eau ou eau-dans-huile, d'un gel aqueux ou huileux, d'une dispersion de vésicules notamment lipidiques. Cette composition peut être plus ou moins fluide et avoir l'aspect d'une crème, d'une pommade, d'un lait, d'une lotion, d'une pâte, d'une mousse. Cette composition peut éventuellement être appliquée sur la peau sous forme d'aérosol.

La présente invention se rapporte également à un procédé cosmétique et/ou dermatologique de dépigmentation et/ou de blanchiment de la peau consistant à appliquer sur la peau une composition comprenant l'acide L-2-oxothiazolidine 4-carboxylique.

L'invention va maintenant être illustrée à l'aide des exemples qui suivent. Les concentration sont données en pourcentage en poids.

| **Exemple 1 : Crème blanchissante pour le visage** | |
|---|---|
| - Acide L-2-oxothiazolidine 4-carboxylique | 2 |
| - Stéarate de sodium | 3 |
| - Huile de vaseline | 6 |
| - Alkyl paraben | 0,05 |
| - Sorbate de potassium | 10 |
| - Alcool stéarylique | 1 |
| - Parfum | 1 |
| - Eau | qsp 100 |

| **Exemple 2 : Crème blanchissante pour le corps** | |
|---|---|
| - Acide L-2-oxothiazolidine 4-carboxylique | 5 |
| - Huile de jojoba | 13 |
| - Cire de sipol | 6 |
| - Palmitate d'isopropyle | 2 |
| - Glycérol | 15 |
| - Alkyl paraben | 0,5 |
| - Parfum | 1 |
| - Eau | qsp 100 |

## Revendications

1. Utilisation de l'acide L-2-oxothiazolidine 4-carboxylique dans une composition cosmétique comme agent dépigmentant et/ou blanchissant de la peau.

2. Utilisation de l'acide L-2-oxothiazolidine 4-carboxylique pour la fabrication d'une composition dermatologique destinée à la dépigmentation et/ou au blanchiment de la peau.

3. Utilisation selon la revendication 1 ou 2, caractérisée en ce que l'acide L-2-oxothiazolidine 4-carboxylique est présent en une quantité allant de 0,1 à 10% en poids par rapport au poids total de la composition.

4. Utilisation selon la revendication précédente, caractérisée en ce que que l'acide L-2-oxothiazolidine 4-carboxylique est présent en une quantité allant de 2 à 5 % en poids par rapport au poids total de la composition.

5. Utilisation selon la revendication 1 ou 2, caractérisée en ce que la composition comprend de plus au moins un ingrédient choisi parmi les corps gras, les conservateurs, les vitamines, les gélifiants, les parfums, les tensioactifs, l'eau, les antioxydants, les charges, les filtres, les hydratants et leurs mélanges.

6. Utilisation selon la revendication précédente, caractérisée en ce que le corps gras est choisi parmi les huiles et les cires.

7. Utilisation selon la revendication 5, caractérisée en ce que le tensioactif est choisi parmi le mono diglycéryl stéarate de sodium, le stéarate de sodium.

8. Utilisation selon la revendication 5, caractérisée en ce que le gélifiant est choisi parmi les polyéthylènes glycol éventuellement oxyéthylénés.

9. Procédé cosmétique de dépigmentation et/ou blanchiment de la peau, caractérisé en ce qu'il consiste à appliquer sur la peau une composition comprenant l'acide L-2-oxothiazolidine 4-carboxylique.

## Claims

1. Use of L-2-oxothiazolidine-4-carboxylic acid in a cosmetic composition as an agent for depigmenting and/or bleaching the skin.

2. Use of L-2-oxothiazolidine-4-carboxylic acid for manufacturing a dermatological composition intended for depigmenting and/or bleaching the skin.

3. Use according to Claim 1 or 2, characterized in that the L-2-oxothiazolidine-4-carboxylic acid is present in an amount ranging from 0.1 to 10% by weight relative to the total weight of the composition.

4. Use according to the preceding claim, characterized in that the L-2-oxothiazolidine-4-carboxylic acid is present in an amount ranging from 2 to 5% by weight relative to the total weight of the composition.

5. Use according to Claim 1 or 2, characterized in that the composition furthermore comprises at least one ingredient chosen from fatty substances, preserving agents, vitamins, gelling agents, fragrances, surfactants, water, antioxidants, fillers, screening agents and moisturizers, and mixtures thereof.

6. Use according to the preceding claim, characterized in that the fatty substance is chosen from oils and waxes.

7. Use according to Claim 5, characterized in that the surfactant is chosen from sodium mono diglyceryl stearate and sodium stearate.

8. Use according to Claim 5, characterized in that the gelling agent is chosen from polyethylene glycols which are optionally oxyethylenated.

9. Cosmetic process for depigmenting and/or bleaching the skin, characterized in that it consists in applying to the skin a composition comprising L-2-oxothiazolidine-4-carboxylic acid.

## Patentansprüche

1. Verwendung von L-2-Oxothiazolidin-4-carbonsäure in einer kosmetischen Zusammensetzung als Mittel zum Depigmentieren und/oder Bleichen der Haut.

2. Verwendung von L-2-Oxothiazolidin-4-carbonsäure zur Herstellung einer dermatologischen Zusammensetzung, die zum Depigmentieren und/oder Bleichen der Haut bestimmt ist.

3. Verwendung nach Anspruch 1 oder 2, dadurch gekennzeichnet, daß die L-2-Oxothiazolidin-4-carbonsäure in einem Mengenanteil von 0,1 bis 10 Gew.-%, bezogen auf das Gesamtgewicht der Zusammensetzung, vorliegt.

4. Verwendung nach dem vorhergehenden Anspruch, dadurch gekennzeichnet, daß die L-2-Oxothiazolidin-4-carbonsäure in einem Mengenanteil von 2 bis 5 Gew.-%, bezogen auf das Gesamtgewicht der Zusammensetzung, vorliegt.

5. Verwendung nach Anspruch 1 oder 2, dadurch gekennzeichnet, daß die Zusammensetzung ferner mindestens einen Inhaltsstoff enthält, der unter den Fettsubstanzen, Konservierungsmitteln, Vitaminen, Gelbildnern, Parfums, grenzflächenaktiven Stoffen, Wasser, Antioxidantien, Füllstoffen, Filtern, Hydratisierungsmitteln und deren Gemischen ausgewählt ist.

6. Verwendung nach dem vorhergehenden Anspruch, dadurch gekennzeichnet, daß die Fettsubstanz unter den Ölen und Wachsen ausgewählt ist.

7. Verwendung nach Anspruch 5, dadurch gekennzeichnet, daß der grenzflächenaktive Stoff unter Monodiglycerinstearat von Natrium und Natriumstearat ausgewählt ist.

8. Verwendung nach Anspruch 5, dadurch gekennzeichnet, daß der Gelbildner unter den gegebenenfalls ethoxylierten Polyethylenglykolen ausgewählt ist.

9. Kosmetisches Verfahren zum Depigmentieren und/oder Bleichen der Haut, dadurch gekennzeichnet, daß es darin besteht, auf die Haut eine Zusammensetzung aufzutragen, die L-2-Oxothiazolidin-4-carbonsäure enthält.
